(19) Europäisches Patentamt
European Patent Office
Office européen des brevets

(11) **EP 4 070 807 A1**

(12) **EUROPEAN PATENT APPLICATION**
published in accordance with Art. 153(4) EPC

(43) Date of publication:
**12.10.2022 Bulletin 2022/41**

(21) Application number: **20897160.6**

(22) Date of filing: **26.11.2020**

(51) International Patent Classification (IPC):
A61K 35/747 (2015.01)     A23K 10/18 (2016.01)
A23K 10/30 (2016.01)      A23L 33/10 (2016.01)
A23L 33/135 (2016.01)     A61K 47/26 (2006.01)
A61K 47/36 (2006.01)      A61K 47/46 (2006.01)
A61P 25/00 (2006.01)      A61P 25/08 (2006.01)
A61P 25/20 (2006.01)      A61P 25/22 (2006.01)
A61P 25/24 (2006.01)      C12N 1/20 (2006.01)

(52) Cooperative Patent Classification (CPC):
A23K 10/18; A23K 10/30; A23L 33/10;
A23L 33/135; A61K 35/747; A61K 47/26;
A61K 47/36; A61K 47/46; A61P 25/00;
A61P 25/08; A61P 25/20; A61P 25/22;
A61P 25/24; C12N 1/20

(86) International application number:
**PCT/JP2020/044055**

(87) International publication number:
**WO 2021/111982 (10.06.2021 Gazette 2021/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.12.2019   JP 2019218218**

(71) Applicant: **Life Quality Institute, Inc.
Tokyo, 101-0053 (JP)**

(72) Inventor: **NAGATA Katsutaro
Tokyo 1010053 (JP)**

(74) Representative: **Osha Liang
2, rue de la Paix
75002 Paris (FR)**

(54) **AGENT FOR PREVENTION OR REMEDIATION OF STRESS DISORDERS AND COMPOSITION CONTAINING SAME**

(57)     The present invention aims to provide an agent for preventing or ameliorating stress disorders, which agent is highly safe and effective, causes less side effects, and can be continuously used for a long time.

One embodiment of the present invention is an agent for preventing or ameliorating stress disorders comprising, as an active ingredient, a lactic acid bacterium Lactobacillus delbrueckii subspecies lactis or a lactic acid bacterium-derived ingredient therefrom.

Figure 1: Changes in CHCW before and after administration

**Description**

TECHNICAL FIELD

The present invention relates to an agent and a composition for preventing, treating or ameliorating stress disorders, and more specifically relates to an agent for preventing, treating or ameliorating stress disorders comprising a lactic acid bacterium and/or a lactic acid bacterium-derived ingredient as an active ingredient, and to a composition comprising the same.

BACKGROUND ART

Stress disorders are physical and/or mental disorders caused by stress. Stress disorders can be classified into the following three groups.

1. Adjustment disorders

Adjustment disorders are conditions of maladjustment which have resulted in mental and/or physical disorders due to inability to adapt to changes in the environment. According to ICD-10 (World Health Organization Diagnostic Guidelines), it is defined as "states in which social function is significantly impaired, due to emotional or behavioral symptoms caused by stress factors." Symptoms of adjustment disorders include emotional symptoms such as anxiety, anger, impatience and tension, and behavioral symptoms such as aggressive behavior, e.g., binge eating, binge drinking and reckless driving, and "acting like a baby" in the case of children. In addition, as physical symptoms, symptoms of autonomic imbalance (sweating, palpitation, dizziness, neurotic reaction, and the like) may be observed. Adjustment disorders may manifest (be diagnosed) as depressive reaction, depression, neurotic reactions, manic depression, anxiety, panic disorder, sleep disorders, epilepsy, schizophrenia, atypical psychosis, and the like.

2. Acute stress disorder

Acute stress disorder is diagnosed based on the criteria such as those recommended in "the Diagnostic and Statistical Manual of Mental Disorders, Fifth Edition" (DSM-5), including intrusive symptoms such as flashbacks, negative mood, dissociative symptoms, avoidance symptoms, and arousal symptoms. Patients have had direct or indirect exposure to a traumatic event, have recurrent recollections of the traumatic event, and present symptoms such as sleep disorders and difficulty concentrating. Acute stress disorder causes significant distress lasting three days or more, but unlike post-traumatic stress disorder (PTSD), it does not persist for one month or more. Patients' symptoms may also manifest as disorders of homeostasis (such as autonomic, endocrine, or immune system-induced psychosomatic disorders, hyperadaptive states, anorexia nervosa, and bulimia nervosa) and mental disorders (such as depressive reaction, neurotic reaction, manic-depressive illness, anxiety, panic disorder, and sleep disorders).

3. Post-traumatic stress disorder (PTSD)

Post-traumatic stress disorder is a maladaptive condition caused by too much unacceptable stress resulting from a fearful, helpless, or frightening event (e.g., combat, sexual assault, natural or man-made disaster, in which self or others are seriously injured or threatened with death) that is directly or indirectly experienced. In post-traumatic stress disorder, symptoms such as nightmares and flashbacks of the traumatic event, emotional numbing, depression, other anxiety disorders, drug abuse, and sleep disorders may be observed and may manifest as a continuation of acute stress disorder. Diagnosis is made clinically based on DSM-5 criteria.

Stress disorders are diagnosed according to the respective diagnostic criteria, mainly by means of a thorough medical interview and psychological tests. There are many types of psychological tests. For example, the "Comprehensive Health Check for Workers" (CHCW; early check of mental and physical health for workers) was developed for early detection of stressful conditions and is also used to diagnose functional pathologies (Non-Patent Document 1, Non-Patent Document 2).

Conventional treatments for stress disorders include counseling (psychoanalysis, cognitive-behavioral therapy, existential therapy, and the like), pharmacotherapy (psychotropic drugs such as antidepressants and anti-anxiety medications, antipsychotic drugs, herbal medicines, and the like), and physical therapy (translocation therapy, spa therapy, and the like).

However, the drugs used in current pharmacotherapy have many side effects, from which patients may suffer. Counseling and physical therapy may be difficult to continue because the financial and time burdens involved are too much. Therefore, safer, more effective, and less burdensome means of treatment for patients is desired.

In recent years, functional lactic acid bacteria have attracted attention in the maintenance and improvement of health, various strains have been isolated and the utility of these strains are being investigated. Lactobacillus delbrueckii subspecies lactis KLAB-4 strain (Lactobacillus delbrueckii subsp. lactis KLAB-4) is a strain isolated from fermented milk, and has been deposited to National Institute of Technology and Evolution (2-5-8, Kazusakamatari, Kisarazu-shi, Chiba, Japan) as a novel lactic acid bacterium belonging to genus Lactobacillus, species delbrueckii, subspecies lactis (Accession No. NITE BP-394; the national deposition strain of August 9, 2007 of the original deposition date was transferred to the International Deposition under the Budapest Treaty (September 22, 2008)). This lactic acid bacterium has been known to have an excellent anti-allergic function, and further an anti-autoimmune disease function, diabetes-improving function, and hypoglycemia-improving function (Patent Documents 1 and 2, Non-Patent Document 3).

PRIOR ART DOCUMENTS

PATENT DOCUMENTS

Patent Document 1: Japanese Patent Gazette No. 5554994
Patent Document 2: Japanese Patent Gazette No. 6747724

NON-PATENT DOCUMENTS

Non-Patent Document 1: "Development of a New Stress Questionnaire "Comprehensive Health Check for Workers" (CHCW)," Akira Tsuda, Teruichi Shimomitsu, Yuko Odagiri, Ayumi Fusejima, Yoshiyuki Tanaka, Hisayoshi Okamura, Hideyo Yamaguchi, Tetsuro Yamamoto, Harald Mori, Alexander Batthyany, Amarendra N. Singh, Katsutaro Nagata, Comprehensive Medicine 11 (1), 2-28 (2012)
Non-Patent Document 2: "Evaluation of fibromyalgia patients by the Comprehensive Health Check for Workers (CHCW)," Katsutaro Nagata, Asano Kondo, Akira Tsuda, Ayumi Fusejima, Jpn J Psychosom Med Vol. 54 No. 11, 1039-1046 (2014)
Non-Patent Document 3: "Health function of lactic acid bacteria (Lactic Acid Bacteria and Human Health)," Airo Tategaki, Comprehensive Medicine, 17 (1), 8-19 (2018)

SUMMARY OF THE INVENTION

PROBLEMS TO BE SOLVED BY THE INVENTION

An object of the present invention is to provide an agent for preventing or ameliorating stress disorders, which agent is highly safe and effective, causes less side effects, and can be continuously used for a long time.

MEANS FOR SOLVING THE PROBLEM

The inventor of the present invention diligently studied to solve the above problem, as a result, found that a specific lactic acid bacterium shows an excellent preventing or ameliorating effect on stress disorders, and completed the present invention.
According to the present invention, the followings are provided:

[1] An agent for preventing or ameliorating stress disorders comprising, as an active ingredient, a lactic acid bacterium Lactobacillus delbrueckii subspecies lactis or a lactic acid bacterium-derived ingredient therefrom;
[2] The agent for preventing or ameliorating stress disorders according to the above described [1], wherein said stress disorders are one or more of adjustment disorders, autonomic imbalance (vegetative dystonia), depressive reaction, depression, neurotic reaction, manic depression, anxiety, panic disorder, sleep disorders, epilepsy, schizophrenia, and atypical psychosis;
[3] The agent for preventing or ameliorating stress disorders according to the above described [1] or [2], wherein said lactic acid bacterium comprises a lactic acid bacterium selected from the group consisting of Lactobacillus delbrueckii subspecies lactis KLAB-4 strain (NITE BP-394) and variants thereof;
[4] The agent for preventing or ameliorating stress disorders according to any one of the above described [1] to [3], which further comprises a water-soluble dietary fiber ingredient, or which is used in combination with a water-soluble dietary fiber ingredient;

[5] The agent for preventing or ameliorating stress disorders according to the above described [4], wherein said water-soluble dietary fiber ingredient is selected from the group consisting of glucomannan, pectin, guar gum, alginic acid, polydextrose, β-glucan, fructan, inulin, levan, graminan, gum arabic, maltitol, psyllium, indigestible oligosaccharide, indigestible dextrin, agarose, sodium alginate, carrageenan, fucoidan, porphyran, laminaran, seaweed, and agar;

[6] The agent for preventing or ameliorating stress disorders according to the above described [5], wherein said water-soluble dietary fiber ingredient is glucomannan;

[7] A composition comprising the agent for preventing or ameliorating stress disorders according to any one of the above described [1] to [6];

[8] A pharmaceutical composition comprising the agent for preventing or ameliorating stress disorders according to any one of the above described [1] to [6];

[9] A food or drink composition comprising the agent for preventing or ameliorating stress disorders according to any one of the above described [1] to [6];

[10] A feed composition for animal or a pharmaceutical composition for animal comprising the agent for preventing or ameliorating stress disorders according to any one of the above described [1] to [6];

[11] A method for preventing or ameliorating stress disorders, comprising administering the agent for preventing or ameliorating stress disorders according to any one of the above described [1] to [6], or the composition according to any one of the above described [7] to [10], to a subject.

EFFECT OF THE INVENTION

The agent for preventing or ameliorating stress disorders of the present invention and the composition comprising the same can effectively ameliorate stress disorders. Since the agent for preventing or ameliorating stress disorders and the composition comprising the same of the present invention comprise a lactic acid bacterium having high safety or a lactic acid bacterium-derived ingredient therefrom as an active ingredient, they are free from worry about side effects and highly safe even after administration or intake for a long term. Further, a water-soluble dietary fiber ingredient, for example, glucomannan is also a beneficial ingredient which human beings have eaten for a long time, and is highly safe. Therefore, the agent for preventing or ameliorating stress disorders of the present invention and the composition comprising the same can be used for prevention and can also be used for treatment.

BRIEF DESCRIPTION OF THE DRAWINGS

[Figure 1]

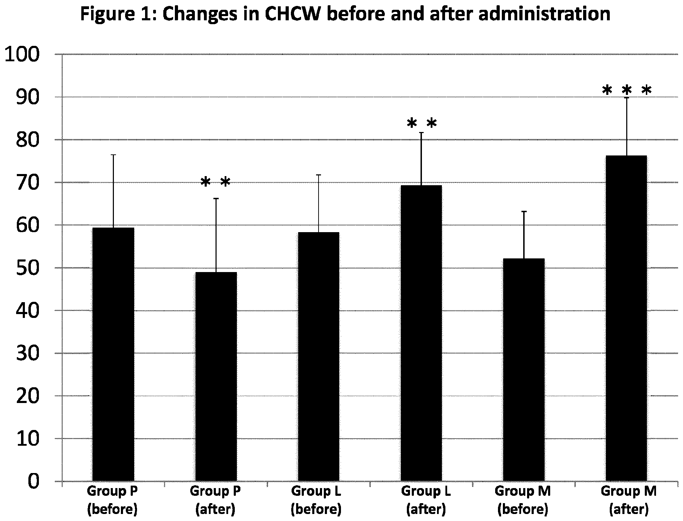

Figure 1 is a figure which compares the values of CHCW before and after administration. The "**" represents a p<0.01 as compared with the value before the administration, and the "***" represents a p<0.001 as compared with the value before the administration.

[Figure 2]

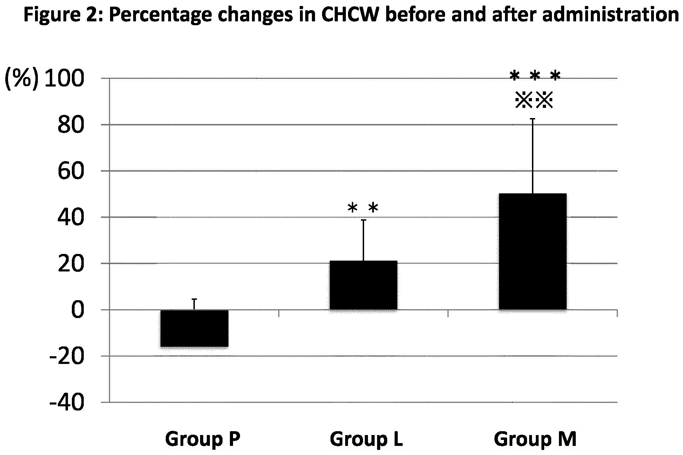

Figure 2 is a figure which shows the percentage changes in CHCW of each group. The "**" represents a p<0.01 as compared with Group P, and the "***" represents a p<0.001 as compared with Group P. The " `※※` represents a p<0.01 as compared with Group L.

MODE FOR CARRYING OUT THE INVENTION

The agent for preventing or ameliorating stress disorders of the present invention comprises, as an active ingredient, a lactic acid bacterium, Lactobacillus delbrueckii subspecies lactis, or a lactic acid bacterium-derived ingredient therefrom. Therefore, the agent of the present invention can comprise only the lactic acid bacterium, Lactobacillus delbrueckii subspecies lactis, or a lactic acid bacterium-derived ingredient therefrom, whereas it can also comprise another active ingredient.

<Lactic acid bacteria>

The lactic acid bacterium, Lactobacillus delbrueckii subspecies lactis which is used in the present invention can be isolated from fermented foods such as fermented milk or cheese, or can be used after obtaining an isolated strain. As the lactic acid bacterium which is used in the present invention, one which contains a lactic acid bacterium selected from the group consisting of Lactobacillus delbrueckii subspecies lactis KLAB-4 strain (Lactobacillus delbrueckii subsp. lactis KLAB-4, hereinafter also referred to as "KLAB-4 strain") and variants thereof is preferred.

KLAB-4 strain is a strain that is described in detail in Patent Document 1 (Japanese Patent Gazette No. 5554994). It has been confirmed that, in this strain, the sequence from the 5'-terminal base to the 544th base of its 16s rRNA gene has a homology of 90% or more to the standard strain of Lactobacillus delbrueckii subsp. lactis, and therefore, it was identified as Lactobacillus delbrueckii subsp. lactis.

The lactic acid bacterium which is preferred for the use in the present invention is not only KLAB-4 strain but also its variant. Such a variant is not especially limited, and can be one which is obtained by natural mutation, one which is obtained by artificially inducing mutation by a known method such as exposure to radiation or mutagen, and the like. As long as the variant has a property (particularly, an action of preventing or ameliorating stress disorders) which is equivalent as compared to KLAB-4 lactic acid bacterium, it can be used in the present invention.

The lactic acid bacterium, Lactobacillus delbrueckii subspecies lactis, for example, KLAB-4 strain or a variant thereof can be cultured by using a known common culture medium and method. These lactic acid bacteria can be cultured by carrying out a usual lactic acid bacterium culture, using a medium in which these cells can grow, for example, MRS medium, under common culture conditions, in a container which is usually used such as a fermentation jar, a test tube, a bottle, a flask, or the like.

The lactic acid bacterium which is used in the present invention may be alive or dead. Live bacteria mean bacterial cells that are alive, and dead bacteria mean bacterial cells that were killed by treatment with heat, pressure, a drug, or the like.

Further, a lactic acid bacterium-derived ingredient which is obtained from a lactic acid bacterium can also be used in the present invention, as with the bacterial cell as long as said ingredient has a comparable function. The lactic acid bacterium-derived ingredient means a part or an ingredient of a bacterial cell, or an ingredient which contains any one of these, and may be, for example, a processed product of bacterial cells which can be obtained by applying at least one of processes such as grinding, crushing, extraction, fractionation, drying, heating, freezing, cooling, concentration, dilution, and the like to the lactic acid bacterium. The lactic acid bacterium-derived ingredient may be in any form such as liquid, paste, powder, granule, or the like. A residue which is obtained after extraction of any ingredient from the bacterial cells may also be used, and for example, a residue after extraction with hot water may be used.

Lactic acid bacterium powder of KLAB-4 strain is commercially available under the product name of "Lactic acid bacterium LAB4" (produced by Kaneka Corporation), and this can also be used.

<Water-soluble dietary fiber ingredient>

The agent for preventing or ameliorating stress disorders of the present invention can comprise a water-soluble dietary fiber ingredient in addition to the lactic acid bacterium or a lactic acid bacterium-derived ingredient therefrom as described above. It was found that the effect of the agent of the present invention can be enhanced by further comprising such an ingredient.

The water-soluble dietary fiber ingredient means a water-soluble dietary fiber, or a food or drug or a food- or drug material containing the same. A specific example of the water-soluble dietary fiber ingredient includes, for example, a natural or synthetic water-soluble dietary fiber or water-soluble dietary fiber-containing material, such as glucomannan, pectin, guar gum, alginic acid, polydextrose, β-glucan, fructan, inulin, levan, graminan, gum arabic, maltitol, psyllium, indigestible oligosaccharide, indigestible dextrin, agarose, sodium alginate, carrageenan, fucoidan, porphyran, laminaran, seaweed and agar.

The water-soluble dietary fiber ingredient can be contained in the agent of the present invention, or can be combined with the lactic acid bacterium or a lactic acid bacterium-derived ingredient therefrom when administered or taken. Alternatively, separate preparations containing each can be administered or taken, and in such a case, both can be administered or taken simultaneously or at intervals. In other words, the agent or composition of the present invention includes not only an embodiment in which the lactic acid bacterium or a lactic acid bacterium-derived ingredient therefrom and a water-soluble dietary fiber are contained in the same composition, but also an embodiment in which they are administered or taken as separate agents or compositions simultaneously or at intervals. For example, the agent of the present invention enveloped in a capsule can be taken with a drink which comprises a water-soluble dietary fiber ingredient.

In case in which a water-soluble dietary fiber ingredient is contained in the agent of the present invention, or in case in which the agent of the present invention and a water-soluble dietary fiber ingredient are used in combination, the ratio (by weight) is not particularly specified, but for example, 0.1 to 2 parts of the water-soluble dietary fiber ingredient is preferred to 1 part of the lactic acid bacterium or a lactic acid bacterium-derived ingredient therefrom.

A mechanism with which the effect of lactic acid bacterium is enhanced by the water-soluble dietary fiber ingredient is not known, and the present invention is not bound by any specific mechanism. For example, glucomannan has a high hydrophilicity, and can absorb about 100 times by weight of water based on the weight of glucomannan. Therefore, it is thought that glucomannan absorbs water in the stomach whereby the volume of glucomannan increases to about 100 times, which leads to a condition where gastric mucosa is covered with a thin membrane. Therefore, it can be thought as one possibility that the presence of the water-soluble dietary fiber ingredient can regulate the absorption of lactic acid bacterium.

<Other ingredients>

The agent for preventing or ameliorating stress disorders of the present invention comprises the lactic acid bacterium or a lactic acid bacterium-derived ingredient therefrom as described above as the only essential ingredient, but it can comprise another active ingredient such as a water-soluble dietary fiber ingredient or it can be used in combination with another active ingredient. The composition which comprises the agent for preventing or ameliorating stress disorders of the present invention comprises one or more other ingredients in addition to the agent of the present invention.

The composition of the present invention can be a pharmaceutical composition, a food or drink composition, a feed composition for animal, a pharmaceutical composition for animal, and the like, and a specific embodiment thereof is not limited. For example, in case in which the composition of the present invention is used as a pharmaceutical composition, as a form or dosage form thereof, a capsule, a tablet, a pill, a powder preparation, a granule preparation, a drink, a syrup, an injection, a transfusion, a nasal drop, an eye drop, a suppository, a plaster, a spray and the like can be exemplified. In case in which the composition of the present invention is used as a food or drink composition, it can be in a form of general food together with various food materials or ingredients, or can be a functional food such as a supplement in a form such as a capsule or a tablet.

An arbitrary ingredient contained in the composition of the present invention can be appropriately selected depending on the property, form, process for preparation, and the like of the composition, and can be various ingredients or additives which are known in the pharmaceutical, food, or cosmetic industry, and the like. As additives which can be contained in the composition of the present invention, an excipient, a disintegrant, a lubricant, a binding agent, a surfactant, a fluidity promoter, a coloring agent, a solvent, a thickener, a dispersing agent, a pH-regulator, a moisturizer, a stabilizer, a preservative, a fragrance or flavor, and the like that are pharmaceutically acceptable, or are usually used in the pharmaceutical, food, or cosmetic industry, and the like, can be exemplified.

These additives are appropriately selected according to the desired dosage form and the like. For example, in case in which the composition of the present invention such as a pharmaceutical composition or a food or drink composition is in the form of a powdered preparation, a granular preparation, a tablet, a capsule, and the like, examples of the excipient which is used include monosaccharides or disaccharides such as lactose, sucrose, glucose, sorbitol and lactitol, starches such as corn starch and potato starch, crystalline cellulose, and inorganic substances such as light silica gel, synthetic aluminum silicate, magnesium metasilicate aluminate, calcium hydrogen phosphate, and silicon dioxide. Furthermore, a binding agent, a disintegrant, a surfactant, a lubricating agent, a fluidity promoter, an antioxidant, an aggregation preventing agent, an absorption promoter, a solubilizer, a stabilizer, a preservative, a desiccant, a coloring agent, a fragrance or flavor, and the like can be appropriately used as necessary.

Examples of the binding agent include starch, dextrin, powdered gum arabic, gelatin, hydroxypropyl starch, carboxymethyl cellulose sodium salt, methyl cellulose, crystalline cellulose, ethyl cellulose, and polyvinylpyrrolidone.

Examples of the disintegrant include starches, carboxymethyl cellulose (CMC), hydroxypropyl cellulose (HPC), carboxymethyl cellulose sodium salt, polyvinylpyrrolidone, and the like.

Examples of the surfactant include soybean lecithin, sucrose fatty acid ester, and the like; examples of the lubricant include talc, wax, sucrose fatty acid esters, hydrogenated vegetable oils, calcium stearate, magnesium stearate, and the like; and examples of the fluidity promotor include anhydrous silicic acid, dry aluminum hydroxide, magnesium silica, and the like.

The above also applies to a case in which the composition of the present invention is used as a feed for animal or a pharmaceutical composition for animal, and the composition of the present invention can be in a form or dosage form such as a feed, a supplement, a medicament, or the like as desired by using various materials for feed or pharmaceutically acceptable ingredients.

The agent or composition of the present invention may be administered or used alone or may be used in combination with other agent(s). Furthermore, in a case in which the agent or composition of the present invention is used in combination with other agent(s), the two may be used simultaneously or may be used one after the other.

The route of administration (or intake or application) of the agent or composition for preventing or ameliorating stress disorders of the present invention can be appropriately selected from, for example, oral, transdermal, transintestinal, intrarectal administration route, and the like. The amount of administration (or intake or application) per day of the agent or composition of the present invention which is effective for prevention or amelioration of stress disorders varies depending on the form of the preparation, the method or route of administration and the like, the age and body weight of the subject, the severity of the disease and the like; for example, in case of oral route, in the human being, generally about 0.1 mg to about 1000 mg/kg body weight/day, preferably about 1 mg to about 500 mg/kg body weight/day, and most preferably about 2 mg to about 300 mg/kg body weight/day, as the amount of the effective ingredient that is a lactic acid bacterium or a lactic acid bacterium-derived ingredient, can be administered or taken all at once or in divisions. The timing of administration or intake is not particularly specified, and for example, can be simultaneous with a meal, just after a meal, just before a meal, before bedtime, or the like.

Since the agent or composition of the present invention is highly safe, it can be administered to not only patients but also healthy subjects. Further, the agent or composition of the present invention can be administered (or taken or applied) similarly to subjects, specifically human being and non-human animals, preferably mammals. Examples of the non-human animals include domestic animals such as cow, horse, pig and sheep, and companion animals such as dog and cat. The amount of administration can be adjusted depending on the characteristics of the animal, using the above amount as the standard. For example, in case of a small animal, the amount of administration or intake can be adjusted and given so that it corresponds to about 0.01 to about 1000 mg/kg body weight /day, more preferably about 0.1 to about 30 mg/kg body weight/day as the amount of the lactic acid bacterium or a lactic acid bacterium-derived ingredient therefrom.

Whether stress disorders have been ameliorated or not by the agent or composition of the present invention can be evaluated by determining whether the number and/or degree of the symptoms has increased or decreased at a later time point as compared to an earlier time point out of different time points such as before and after the administration or intake, or during the administration or intake, or after the administration or intake.

The method of evaluating stress disorders can be by medical examination based on the diagnostic criteria as described above, and the use of the CHCW is advantageous. The CHCW is a questionnaire composed of 30 items in four categories: physical (body), psychological (mind), social (environment), and existential (purpose in life), and is a simple yet statistically sound questionnaire method (Non-Patent Document 1, Non-Patent Document 2). The CHCW enables objective comparison or evaluation of the presence or absence or degree of stress disorders by quantifying them using certain criteria.

EXAMPLES

Hereinafter, the present invention will be more specifically described by way of Examples, but the present invention is not limited by these Examples.

1. Production of capsule preparation

As a lactic acid bacterium Lactobacillus delbrueckii subspecies lactis, KLAB-4 lactic acid bacterium powder (product name: "Lactic acid bacterium LAB4" (manufactured by Kaneka Corporation)), and as a water-soluble dietary fiber ingredient, glucomannan (product name: "RHEOLEX RS" (manufactured by Shimizu Chemical Corporation) were used, and the following 3 kinds of capsule preparations were produced.

Production example 1:

One hundred mg of the lactic acid bacterium powder, 50 mg of glucomannan powder, 17.5 mg of crystalline cellulose powder, 2 mg of calcium stearate powder, and 0.5 mg of silicone dioxide powder per 1 capsule were mixed and stirred until uniform, and then filled into a commercially available capsule (product name: "Pig Gelatin Clear No. 3" (manufactured by Qualicaps Co., Ltd.).

Production example 2:

One hundred mg of the lactic acid bacterium powder, 50 mg of lactose, 17.5 mg of crystalline cellulose powder, 2 mg of calcium stearate powder, and 0.5 mg of silicone dioxide powder per 1 capsule were mixed and stirred until uniform, and then filled into the same commercially available capsule as that used in Production example 1.

Comparative example:

The same capsule preparation as in Production example 1 was produced except that it contained 150 mg of lactose in place of the lactic acid bacterium powder and glucomannan powder.

These 3 kinds of capsule preparations did not have a difference in the appearance, taste and smell, and were not distinguishable.

2. Study on the effect on stress disorders

The present study was carried out after sufficient informed consent was obtained upon obtaining the approval from the ethical committee of a public interest incorporated foundation, the International Foundation of Comprehensive Medicine (2017; the study period was from December 2017 to June 2019).

<Subjects>

Sixty-four patients who had been diagnosed as stress disorders by a medical specialist (32 men and 32 women) were divided into 3 groups of placebo group (P), lactic acid bacterium group (L) and lactic acid bacterium + glucomannan group (M) by an enveloped method to investigate the effect of the agent of the present invention by a double blind method.

There was no statistically significant difference in the age and the sex of the subjects among the groups. The details of the subjects constituting each group are shown in Table 1.

Table 1: Details of subjects of each group

|         | Men | Women | Total | Age          |
|---------|-----|-------|-------|--------------|
| Group P | 11  | 12    | 23    | 34.5 + 9.6   |
| Group L | 10  | 10    | 20    | 35.2 + 9.2   |
| Group M | 11  | 10    | 21    | 35.6 + 13.1  |
| Total   | 32  | 32    | 64    | 35.1 + 10.5  |

Group P, Group L, and Group M took 3 capsules of Comparative example, Production example 2, and Production example 1, respectively, per day (1 capsule at a time) immediately after meals. The administration period was 3 months.

<Evaluation of stress disorders>

The evaluation was performed by the CHCW. Each subject was asked to complete the CHCW before the start and after the end of administration, and changes were compared. To protect personal information, individual names in the questionnaire were erased and indicated by numbers.

The scores for all items were summed to produce the total numbers for evaluation. The 30 items in the CHCW were each rated on the scale from 1 to 5, with 5 being "best" and 1 being "worst." Thus, a score of 150 is the highest, which reflects a condition with no stress at all, and a score of 30 is the lowest, which is evaluated as the most stressful condition. Stress disorders are diagnosed comprehensively with a score of 82 or less.

The statistical figures were expressed as an average value + standard deviation, and analyzed by a statistic software SSMC, MAC version.

The results are shown in Figures 1 and 2.

Figure 1 is a figure which compares the changes in the CHCW scored before the start and after the end of administration. There were no statistically significant differences in the scores among groups before administration. Group P showed a significant worsening after administration. On the other hand, Group L and Group M showed a significant improvement after administration compared to before administration. When Group L (**: $p<0.01$) and Group M (***: $p<0.001$) were compared, Group M showed a larger significant difference.

Figure 2 is a figure which compares the percent changes in the CHCW for all groups before and after administration. The percent changes in the CHCW were calculated as follows:

```
Percent change (%) = [(CHCW after administration - CHCW before
administration) / CHCW before administration] x 100.
```

As shown in Figure 2, compared to Group P, Group L (**: $p<0.01$) and Group M (***: $p<0.001$) showed a significant increase in the CHCW. When Group L and Group M were compared, Group M showed a significant increase in the CHCW compared to Group L ( ※:※ $p<0.01$).

investigation of safety>

In each group, blood and urine specimens were collected before the start and after the end of the administration, and complete blood count (red blood cell, white blood cell, hemoglobin, hematocrit, and platelet), Alb, GOT, GPT, LDH, AIP, y-GTP, amylase, BUN, creatinine, blood sugar, hemoglobin A1c, and urine (protein, sugar, urobilinogen) were tested to compare the results.

Any group did not exhibit any abnormalities in the blood and urine tests before and after the administration. As a side effect, loose stool was observed in 2 cases in Group L, and in 1 case in Group M.

According to the results of the present study, stress disorders were improved in both of Group L and Group M that were administered the agent for preventing or ameliorating stress disorders of the present invention. That is, Group L and Group M showed significant efficacy to stress disorders as compared with Group P. When Group L and Group M were compared, Group M showed a greater efficacy. As the side effects, only slight soft stool was observed in a few subjects in both Group L and Group M, which demonstrated that the agent or composition of the present invention is highly safe.

The present application is based on the Japanese Patent Application No. 2019-218218 filed on December 2, 2019, and the subject matters described in the specification and the scope of the claims for patent of Japanese Patent Application No. 2019-218218 are all incorporated in this specification.

## Claims

1. An agent for preventing or ameliorating stress disorders comprising, as an active ingredient, a lactic acid bacterium Lactobacillus delbrueckii subspecies lactis or a lactic acid bacterium-derived ingredient therefrom.

2. The agent for preventing or ameliorating stress disorders according to claim 1, wherein said stress disorders are one or more of adjustment disorders, autonomic imbalance (vegetative dystonia), depressive reaction, depression, neurotic reaction, manic depression, anxiety, panic disorder, sleep disorders, epilepsy, schizophrenia, and atypical psychosis.

3. The agent for preventing or ameliorating stress disorders according to claim 1 or 2, wherein said lactic acid bacterium comprises a lactic acid bacterium selected from the group consisting of Lactobacillus delbrueckii subspecies lactis KLAB-4 strain (NITE BP-394) and variants thereof.

4. The agent for preventing or ameliorating stress disorders according to any one of claims 1 to 3, which further comprises a water-soluble dietary fiber ingredient, or which is used in combination with a water-soluble dietary fiber ingredient.

5. The agent for preventing or ameliorating stress disorders according to claim 4, wherein said water-soluble dietary fiber ingredient is selected from the group consisting of glucomannan, pectin, guar gum, alginic acid, polydextrose, β-glucan, fructan, inulin, levan, graminan, gum arabic, maltitol, psyllium, indigestible oligosaccharide, indigestible dextrin, agarose, sodium alginate, carrageenan, fucoidan, porphyran, laminaran, seaweed, and agar.

6. The agent for preventing or ameliorating stress disorders according to claim 5, wherein said water-soluble dietary fiber ingredient is glucomannan.

7. A composition comprising the agent for preventing or ameliorating stress disorders according to any one of claims 1 to 6.

8. A pharmaceutical composition comprising the agent for preventing or ameliorating stress disorders according to any one of claims 1 to 6.

9. A food or drink composition comprising the agent for preventing or ameliorating stress disorders according to any one of claims 1 to 6.

10. A feed composition for animal or a pharmaceutical composition for animal comprising the agent for preventing or ameliorating stress disorders according to any one of claims 1 to 6.

11. A method for preventing or ameliorating stress disorders, comprising administering the agent for preventing or ameliorating stress disorders according to any one of claims 1 to 6, or the composition according to any one of claims 7 to 10, to a subject.

## Figure 1: Changes in CHCW before and after administration

## Figure 2: Percentage changes in CHCW before and after administration

| | **INTERNATIONAL SEARCH REPORT** | International application No. |
| | | PCT/JP2020/044055 |

**A. CLASSIFICATION OF SUBJECT MATTER**

Int.Cl. A61K35/747(2015.01)i, A23K10/18(2016.01)i, A23K10/30(2016.01)i, A23L33/10(2016.01)i, A23L33/135(2016.01)i, A61K47/26(2006.01)i, A61K47/36(2006.01)i, A61K47/46(2006.01)i, A61P25/00(2006.01)i, A61P25/08(2006.01)i, A61P25/20(2006.01)i, A61P25/22(2006.01)i, A61P25/24(2006.01)i, C12N1/20(2006.01)i
FI: A61K35/747, A61P25/22, A61P25/24, A61P25/20, A61P25/00, A61K47/36, A61K47/26, A61K47/46, A23L33/135, A23L33/10, C12N1/20E, A23K10/18, A23K10/30, A61P25/08

According to International Patent Classification (IPC) or to both national classification and IPC

**B. FIELDS SEARCHED**

Minimum documentation searched (classification system followed by classification symbols)
Int.Cl. A61K35/747, A23K10/18, A23K10/30, A23L33/10, A23L33/135, A61K47/26, A61K47/36, A61K47/46, A61P25/00, A61P25/08, A61P25/20, A61P25/22, A61P25/24, C12N1/20

Documentation searched other than minimum documentation to the extent that such documents are included in the fields searched
Published examined utility model applications of Japan      1922–1996
Published unexamined utility model applications of Japan    1971–2020
Registered utility model specifications of Japan            1996–2020
Published registered utility model applications of Japan    1994–2020

Electronic data base consulted during the international search (name of data base and, where practicable, search terms used)
JSTPlus/JMEDPlus/JST7580(JDreamIII), CAplus/REGISTRY/MEDLINE/EMBASE/BIOSIS/WPIDS(STN)

**C. DOCUMENTS CONSIDERED TO BE RELEVANT**

| Category* | Citation of document, with indication, where appropriate, of the relevant passages | Relevant to claim No. |
|---|---|---|
| X | WO 2009/066681 A1 (KANEKA CORPORATION) 28 May 2009 | 7–10 |
| A | (2009-05-28), claims | 1-6, 11 |

☐ Further documents are listed in the continuation of Box C.      ☒ See patent family annex.

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 14 December 2020 | 12 January 2021 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

INTERNATIONAL SEARCH REPORT
Information on patent family members

International application No.

PCT/JP2020/044055

```
WO 2009/066681 A1  28 May 2009   EP 2223697 A1
                                 claims
                                 US 2010/0278795 A1
```

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5554994 B
- JP 6747724 B
- JP 2019218218 A

**Non-patent literature cited in the description**

- the Diagnostic and Statistical Manual of Mental Disorders
- **AKIRA TSUDA ; TERUICHI SHIMOMITSU ; YUKO ODAGIRI ; AYUMI FUSEJIMA ; YOSHIYUKI TANAKA ; HISAYOSHI OKAMURA ; HIDEYO YAMAGUCHI ; TETSURO YAMAMOTO ; HARALD MORI ; ALEXANDER BATTHYANY.** Development of a New Stress Questionnaire ''Comprehensive Health Check for Workers'' (CHCW). *Comprehensive Medicine,* 2012, vol. 11 (1), 2-28
- **KATSUTARO NAGATA ; ASANO KONDO ; AKIRA TSUDA ; AYUMI FUSEJIMA.** Evaluation of fibromyalgia patients by the Comprehensive Health Check for Workers (CHCW). *Jpn J Psychosom Med,* 2014, vol. 54 (11), 1039-1046
- **AIRO TATEGAKI.** Health function of lactic acid bacteria (Lactic Acid Bacteria and Human Health). *Comprehensive Medicine,* 2018, vol. 17 (1), 8-19